Europäisches Patentamt

European Patent Office

Office européən des brevets

(19)

(11) Veröffentlichungsnummer : **0 012 972**
**B1**

# EUROPÄISCHE PATENTSCHRIFT

(12)

(45) Veröffentlichungstag der Patentschrift :
**11.11.81**

(21) Anmeldenummer : **79105222.8**

(22) Anmeldetag : **17.12.79**

(51) Int. Cl.³ . **C 07 C178/00, C 07 C179/10,
C 07 C179/12**

(54) **Verfahren zur Herstellung von Percarbonsäurelösungen.**

(30) Priorität : 29.12.78 DE 2856665

(43) Veröffentlichungstag der Anmeldung :
**09.07.80 (Patentblatt 80/14)**

(45) Bekanntmachung des Hinweises auf die Patenterteilung : **11.11.81 Patentblatt 81/45**

(84) Benannte Vertragsstaaten :
**BE DE FR GB NL SE**

(56) Entgegenhaltungen :
**DE - A - 2 141 155**
**DE - A - 2 141 156**
**DE - A - 2 145 602**
**DE - A - 2 145 603**
**DΞ - A - 2 262 970**
**DE - A - 2 519 288**
**DE - A - 2 519 289**
**DE - A - 2 519 290**
**DE - A - 2 519 296**
**DE - A - 2 519 297**
**DE - A - 2 519 298**

(73) Patentinhaber : **BAYER AG**
**Zentralbereich Patente, Marken und Lizenzen**
**D-5090 Leverkusen 1, Bayerwerk (DE)**

**Degussa Aktiengesellschaft**
**Weissfrauenstrasse 9**
**D-6000 Frankfurt am Main 19 (DE)**

(72) Erfinder : **Goedecke, Ralf, Dr.**
**Ulmenstrasse 10**
**D-6458 Rodenbach 1 (DE)**
Erfinder : **Hofen, Willi**
**Südring 54**
**D-6458 Rodenbach (DE)**
Erfinder : **Seifert, Hermann, Dr.**
**Ruwergasse 4**
**D-5000 Koeln 80 (DE)**
Erfinder : **Siekmann, Gerd, Dr.**
**Wolfskaul 8**
**D-5000 Koeln 80 (DE)**

## Verfahren zur Herstellung von Percarbonsäurelösungen

Die vorliegende Erfindung betrifft ein kontinuierliches Verfahren zur technischen Herstellung von reinen Percarbonsäurelösungen in organischen Lösungsmitteln. Insbesondere betrifft die vorliegende Erfindung ein Verfahren zur Gewinnung von organischen Lösungen von Percarbonsäuren mit 2 bis 5 Kohlenstoffatomen, die frei oder weitgehend frei von Wasser, Wasserstoffperoxid und starker Säure sind.

Percarbonsäuren gewinnen zunehmend an Bedeutung als in technischem Maßstab verwendbare Oxidationsmittel. Mit Percarbonsäuren können beispielsweise Olefine in Epoxide und cyclische Ketone in Lactone umgewandelt werden (vgl. D. Swern, Organic Peroxides, Vol. II, S. 355-502, John Wiley-Interscience 1971, sowie Houben-Weyl, Methoden der Organischen Chemie, Band VI/2, S. 707-711, Georg Thieme Verlag 1963).

Die leicht, z.B. nach dem Verfahren der Deutschen Patentschrift 1 165 576 zugänglichen wäßrigen Percarbonsäurelösungen sind wegen der Anwesenheit von Wasser für die Durchführung derartiger Oxidationen jedoch nicht vorteilhaft, da das Wasser sehr schnell mit den gewünschten Oxidationsprodukten, den Epoxiden oder den Lactonen, weiterreagiert, was zu einer erheblichen Nebenproduktbildung führen kann.

Den gleichen negativen Einfluß auf die Ausbeute an Oxidationsprodukten üben starke Säuren oder aber auch vorhandenes freies Wasserstoffperoxid aus, wobei insbesondere Spuren von Mineralsäuren, die mit den Percarbonsäurelösungen in das Reaktionsgemisch miteingebracht werden, bei gleichzeitiger Anwesenheit von Wasser die erfolgreiche Durchführung der beispielsweise zu Epoxiden oder Lactonen führenden Reaktion gänzlich in Frage stellen können, da die Bildung der Nebenprodukte zur Hauptreaktion wird (vgl. Methodicum Chimicum, Band 5, Seite 170, Georg Thieme Verlag (1975), sowie Houben Weyl, Band VI/2, S. 708, Georg Thieme Verlag (1963) und J. Amer. chem. Soc. *80*, 4079 (1958). Wasserfreie oder weitgehend wasserfreie Lösungen von Percarbonsäuren in organischen Lösungsmitteln können, wie das Studium des Standes der Technik zeigt, auf sehr unterschiedliche Weise gewonnen werden.

Die Methode, Aldehyde durch Oxidation in einem organischen Lösungsmittel in Percarbonsäure umzuwandeln, beispielsweise nach dem Verfahren der DE-AS 1 043 315 (USA-PS 2 804 473), hat den Nachteil, daß während der Durchführung des Verfahrens hochexplosible Zwischenprodukte auftreten (vgl. Ullmanns Encyklopädie der techn. Chemie, Neue Verfahren (Ergänzungsband) S. 181 (1970), und daß die den Aldehyden entsprechenden Carbonsäuren nach erfolgter Oxidationsreaktion der Percarbonsäuren als Nebenprodukte anfallen, was eine Anwendung derartiger Verfahren in größerem Maßstab sowohl aus sicherheitstechnischen als auch aus ökonomischen Gründen weitgehend ausschließt. Die dem Aldehyd entsprechende Carbonsäure kann nicht in das Verfahren zurückgeführt werden. Außerdem enthalten die nach dem Aldehyd-Oxydations-Verfahren gewonnenen organischen Lösungen von Percarbonsäuren Verunreinigungen, die durch den bei der Herstellung gleichzeitig ablaufenden, weiterführenden oxidativen Abbau des Aldehyds bedingt sind.

Weiterhin kann man wasserfreie oder weitgehend wasserfreie, organische Lösungen von Percarbonsäuren durch diskontinuierliche oder kontinuierliche Extraktion und azeotrope Nachentwässerung der anfallenden Extrakte oder aber auch nur allein durch azeotrope Entwässerung der wäßrigen Percarbonsäurelösungen herstellen, wobei man einen Teil des angewendeten organischen Lösungsmittels zum Verdünnen der Percarbonsäure benutzt, wie es in den belgischen Patentschriften 788 727, 788 729 und vor allem 788 728 vorgeschlagen worden ist. Die zu extrahierenden Lösungen werden durch Umsetzen von $H_2O_2$ mit Carbonsäuren in Gegenwart saurer Katalysatoren hergestellt. Die beschriebenen Verfahren sind energieaufwendig, weil in jedem Fall bei der Herstellung der wäßrigen Percarbonsäurelösungen Wasser und zusätzlich die Percarbonsäure destilliert werden müssen. Das stellt eine Maßnahme dar, die einen hohen Energieaufwand erfordert und außerdem wegen der thermischen Belastung der Percarbonsäuren aufwendige Sicherheitsmaßnahmen erfordert. Zudem sind wegen der korrosiven Eigenschaften destillierender Persäuren spezielle Werkstoffe einzusetzen, die ebenfalls eine großtechnische Anwendung dieser Verfahren weitgehend ausschließen.

Das in den auf destillativem Wege gewonnenen, wäßrigen Percarbonsäuren enthaltene Wasser muß, wenn man zu reinen, organischen Lösungen von Persäuren gelangen will, zudem nochmals durch Azeotropdestillation abgetrennt werden, was zusätzliche Maßnahmen erfordert (vgl. Ullmann, Enzyklopädie der Technischen Chemie, Ergänzungsband 1970, Neue Verfahren, S. 181 ff., sowie D. Swern, Organic Peroxides, Vol. I, 1970, S. 313 ff.).

Es ist weiterhin bekannt, daß man organische Percarbonsäurelösungen erhalten kann, wenn man aus einem vorher ins Gleichgewicht gebrachten Gemisch der durch Säuren katalysierten Reaktion

$$\text{Carbonsäure} + H_2O_2 \rightleftarrows \text{Percarbonsäure} + H_2O$$

die Percarbonsäure mit einem mit Wasser nicht mischbaren organischen Lösungsmittel extrahiert.

Dabei wird die Extraktion zur Gewinnung der organischen Lösungen von Percarbonsäuren einstufig, wie in der Deutschen Offenlegungsschrift 1 618 625 beschrieben, oder auch mehrstufig, wie der Deutschen Auslegeschrift 1 048 569 zu entnehmen ist, durchgeführt. Als Katalysator für die Einstellung des obengenannten Gleichgewichtes wird in der technischen Durchführung meist Schwefelsäure benutzt, wie es in der Deutschen Patentschrift 2 262 970 und der Deutschen Patentschrift 2 602 776 sowie in der DE-AS 2 519 289 beschrieben wird. Die Extraktion selbst wird im Gegenstrom, meist in mit den verschiedensten Einbauten versehenen Extraktionssäulen, durchgeführt.

Bei allen diesen neueren Verfahren zur Herstellung von organischen Lösungen von Percarbonsäuren — den sogenannten Extraktionsverfahren — bei denen wäßrige Gemische bestehend aus Percarbonsäure, saurem Katalysator und Wasserstoffperoxid mit organischen Lösungsmitteln extrahiert werden, werden Extrakte erhalten, die noch — teilweise in erheblichem Umfang — Wasser, $H_2O_2$ und starke Mineralsäure enthalten. Die Konzentrationen, mit denen diese Verunreinigungen in der organischen Lösung der Percarbonsäure vorliegen, sind im wesentlichen von zwei Größen abhängig. Zum einen spielt die Polarität des verwendeten organischen Lösungsmittels eine Rolle, zum anderen sind die Extraktionsbedingungen von Einfluß, wie insbesondere die Temperatur, unter der die Extraktion durchgeführt wird, sowie die Konzentration an starker Säure, die in der wäßrigen Phase vorliegt.

Es ist verständlich, daß die Qualität dieser durch Extraktion gewonnenen Percarbonsäurelösungen für die beispielsweise zu Olefinoxiden führende Umsetzung mit Olefinen völlig unzureichend sein muß. Zur Reinigung derartiger organischer Percarbonsäurelösungen sind daher weitere verfahrenstechnische Schritte vorgeschlagen worden.

In der Deutschen Patentschrift 2 262 970, 3. Spalte, Zeile 26-32, wird vorgeschlagen, die Percarbonsäurelösung in einer kontinuierlich durchgeführten Nachextraktion mit Wasser oder einer wäßrigen Lösung im Gegenstrom zu behandeln, um insbesondere das Wasserstoffperoxid zurückzugewinnen, d.h. den Extrakt von in ihm enthaltenen $H_2O_2$ zu reinigen. Der Wassergehalt der so gewaschenen organischen Percarbonsäurelösungen beträgt dann zwischen 1 bis 2 Gew.-%.

Nach dem Verfahren der Deutschen Offenlegungsschrift 2 602 776 wird die Nachbehandlung des rohen Percarbonsäureextraktes mit Schwefelsäure vorgenommen, wobei der hierfür angewendete Extraktor in das zur Gewinnung der Percarbonsäurelösung dienende Extraktionssystem integriert ist. Bedingt durch die Verwendung von wäßriger Schwefelsäure für die Nachwäsche muß die gewaschene Percarbonsäurelösung zwangsläufig neben Wasser auch geringe Anteile an dieser starken Mineralsäure enthalten. Die Gehalte an diesen beiden Verunreinigungen sind aus der genannten Offenlegungsschrift 2 602 776 nicht genau ersichtlich. Jedoch liegen beispielsweise Gehalte an Wasser von 2 Gew.-% und an Schwefelsäure von nur 500 ppm in der organischen Lösung der Percarbonsäure durchaus im Bereich der Realität. Derartige Konzentrationen an diesen Verunreinigungen aber bewirken bei der Herstellung von Epoxiden, wie beispielsweise Propylenoxid, eine erhebliche Nebenproduktbildung, insbesondere auch bei der nachfolgend notwendig werdenden destillativen Aufarbeitung des Epoxid enthaltenden Reaktionsgemisches, so daß die Wirtschaftlichkeit eines mit dieser Persäurelösung arbeitenden Verfahrens in Frage gestellt wird.

Die genannten Nachteile hinsichtlich des Gehaltes an starker Säure und an Wasser in der organischen Percarbonsäurelösung werden beim Verfahren der Deutschen Offenlegungsschrift 2 519 298 weitgehend vermieden. In dieser Offenlegungsschrift wird ein Verfahren zur Herstellung von Propylenoxid beschrieben, wobei als Oxydationsmittel bevorzugt eine benzolische Perpropionsäurelösung eingesetzt wird, die zur Entfernung von starker Säure und Wasserstoffperoxid mit Wasser oder mit einer wäßrigen Lösung gewaschen und nachfolgend zur Entfernung des durch den Waschvorgang in die benzolische Lösung eingebrachten Wassers einer Azeotropdestillation unterworfen wird. Das gelöste und auch das mit der organischen Phase bei der Nachwäsche mitgerissene Wasser wird bei diesem Verfahrensschritt als Wasser-Benzol-Azeotrop in einer Destillationseinheit über Kopf genommen, und das nach Kondensation der Kopfdämpfe und Phasentrennung als leichte Phase anfallende Benzol wird vollständig als Rücklauf auf die Destillationskolonne gegeben.

Dieses Verfahren erfordert einen erheblichen Aufwand an Energie, da beträchtliche Mengen an Wasser abdestilliert werden müssen und zudem große Rücklaufverhältnisse notwendig sind. Außerdem sind die hinsichtlich des Gehaltes an freiem Wasserstoffperoxid in der organischen Lösung nach diesem Verfahren erzielbaren Ergebnisse noch nicht als ausreichend anzusehen.

Demgegenüber wurde nun überraschenderweise gefunden, daß man in technisch und wirtschaftlich vorteilhafter Weise reine organische Lösungen von Percarbonsäuren mit 2 bis 5 Kohlenstoffatomen kontinuierlich durch

a) Umsetzung von wäßrigem Wasserstoffperoxid mit 2 bis 5 Kohlenstoffatome enthaltenden Carbonsäuren in Gegenwart saurer Katalysatoren bei einem Einsatzmolverhältnis von $H_2O_2$ zu Carbonsäure wie 0,5 bis 30 : 1,

b) Gewinnung eines im wesentlichen die Percarbonsäure sowie gegebenenfalls die entsprechende, in Stufe (a) nicht umgesetzte Carbonsäure enthaltenden Extraktes durch Extraktion der gemäß Stufe (a) erhaltenen Reaktionsmischung mit einem organischen Lösungsmittel,

c) Behandlung des Extraktes in einer mit ein bis zehn theoretischen Trennstufen versehenen Extraktionseinheit mit Wasser oder einer wäßrigen Lösung und

d) Entwässerung der gemäß (c) erhaltenen organischen Lösung der Percarbonsäure, gegebenenfalls nach Zusatz eines weiteren organischen Lösungsmittels, durch azeotrope Destillation,

herstellen kann, wenn man die bei der Entwässerung gemäß (d) anfallende organische Phase des Kopfprodukts der Azeotropdestillation ganz oder teilweise in die Stufe (c) und/oder ganz oder teilweise in die Stufe (b) zurückführt. Dabei kann man die gesamte Menge oder einen Teil der nach Stufe (c) zurückzuführenden organischen Phase vorzugsweise der für Schritt (c) verwendeten Extraktionseinheit vor der ersten theoretischen Trennstufe zuführen oder an einer Stelle in die für Stufe (c) benutzte Extraktionseinheit eingeben, die sich hinter der ersten und vor der letzten theoretischen Trennstufe befindet.

Man kann aber auch die gesamte Menge oder einen Teil der bei Stufe (d) anfallenden organischen Phase der Stufe (b) wieder zuführen. Ebenso ist es möglich, die Gesamtmenge der bei Durchführung der Stufe (d) anfallenden organischen Phase oder Teile davon sowohl der Stufe (c) als auch der Stufe (b) zuzuführen.

Als organische Lösungsmittel für das erfindungsgemäße Verfahren eignen sich alle gegenüber der Percarbonsäure inerten Verbindungen, die einen Siedepunkt bei Normaldruck von 30 °C bis 140 °C besitzen. Weiterhin sollen die organischen Lösungsmittel eine möglichst geringe Löslichkeit in Wasser oder wäßriger Schwefelsäure besitzen und darüber hinaus ein Azeotrop mit Wasser bilden können, dessen Siedepunkt mindestens 10 °C unterhalb des Siedepunktes der Percarbonsäure liegt. Es sei angemerkt, daß selbstverständlich die erfindungsgemäß in Stufe (b) und/oder (c) zurückzuführende organische Phase aus (d) im wesentlichen aus organischem Lösungsmittel mit den genannten Kriterien besteht.

Diese Lösungsmittel können sehr unterschiedlicher chemischer Natur sein. Als geeignet erweisen sich beispielsweise aromatische Kohlenwasserstoffe, die sechs bis zehn Kohlenstoffatome enthalten, aliphatische oder cycloaliphatische, jeweils fünf bis zu zwölf Kohlenstoffatome enthaltende Kohlenwasserstoffe, chlorierte chlorierte Kohlenwasserstoffe, die ein bis zehn Kohlenstoffatome sowie ein bis vier Chloratome enthalten, und Ester von ein bis fünf C-Atome enthaltenden Carbonsäuren mit geradkettigen oder verzweigten Alkoholen, in denen ein bis acht C-Atome im Molekül vorliegen, sowie Äther, die zwei bis zehn C-Atome enthalten.

Bevorzugt werden chlorierte Kohlenwasserstoffe verwendet, die sich von Alkanen oder Alkenen ableiten, wie beispielsweise Methylenchlorid, Tetrachlorkohlenstoff, Dichloräthan, 1,2-Dichlorpropan, Tri- und Tetrachloräthylen ; oder aromatische Kohlenwasserstoffe wie Benzol, Toluol oder Chlorbenzol ; oder auch Äther, wie Diisopropyläther und Ester wie Essigsäuremethyl-, Essigsäureäthyl-, Essigsäure-n-propyl- oder - isopropyl- oder Essigsäure-n-butylester. Es ist aber auch möglich und in einigen Fällen sogar vorteilhaft, Gemische dieser Lösungsmittel zu verwenden.

Die säurekatalysierte Umsetzung von $H_2O_2$ mit der Carbonsäure gemäß Stufe (a) kann in an sich bekannter Weise erfolgen, beispielsweise nach den in DE-PS 2 262 970, DE-OS 2 519 298, DE-AS 2 519 289 oder DE-OS 2 602 776 beschriebenen Verfahren. Ebenso werden die Verfahrensschritte (b), (c) und (d) in an sich bekannter Weise durchgeführt, z.B. nach den in DE-PS 2 262 970, DE-OS 2 519 298 oder DE-AS 2 519 289 beschriebenen Verfahren. Als Carbonsäure für die Umsetzung gemäß Stufe (a) kommt beispielsweise Essigsäure, Propionsäure, n-Buttersäure oder Isobuttersäure in Betrackt.

Für den Verfahrensschritt (c) können alle für die Durchführung von Extraktionen geeigneten Apparate Anwendung finden. Im allgemeinen verwendet man nach dem Gegenstromprinzip arbeitende Extraktionskolonnen oder Mischer-Scheider-Batterien, die teilweise im Gegenstrom und teilweise im Kreuzstrom betrieben werden können. Es ist aber auch möglich, verschiedene, kombinierte Extraktoren oder auch Zentrifugal-Extraktionsmaschinen zu verwenden. Man verwendet zweckmäßigerweise 1 bis 18 Vol.-% an Wasser oder wäßriger Lösung, bezogen auf den organischen Extrakt.

Vorzugsweise verwendet man 3 bis 6 Vol.-% Wasser. An Stelle von reinem Wasser kann man auch eine wäßrige Lösung mit mindestens 90 Gew.-% Wasser verwenden, die im wesentlichen frei von Wasserstoffperoxid und von Mineralsäure ist. Zweckmäßig ist es, eine wäßrige Phase zu verwenden, die im Verfahren anfällt. Geeignet ist beispielsweise die wäßrige Phase der Azeotropdestillation gemäß (d).

Für die Stufe (d), die Entwässerung durch azeotrope Destillation, eignen sich die üblichen Kolonnen, wie beispielsweise die bekannten Boden- oder Füllkörperkolonnen. Zweckmäßigerweise werden sowohl der Kolonnenkörper dieser Destillationseinheit als auch die Einbauten sowie die Verdampfereinheit und das Kondensationssystem aus rostfreien Edelstählen gefertigt.

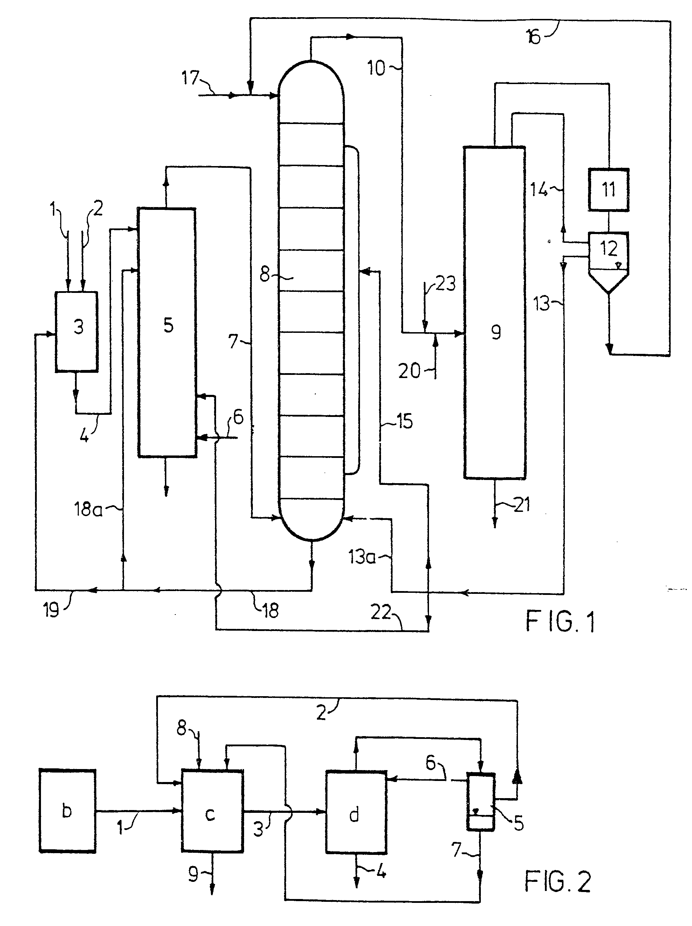

Eine allgemeine Ausführungsform des erfindungsgemäßen Verfahrens sei an Hand des in Figur 1 dargestellten Fließschemas wie folgt beschrieben :

In dem Reaktionssystem (3) wird in an sich bekannter Weise die Umsetzung gemäß Schritt (a) vorgenommen. Über Leitung (1) wird ein Gemisch aus wäßrigem $H_2O_2$ und saurem Katalysator, wie beispielsweise Schwefelsäure, zugeführt, während über (2) die Carbonsäure in das Reaktionssystem gegeben wird. Das die Percarbonsäure enthaltende wäßrige Reaktionsgemisch wird über Leitung (4) dem Extraktionssystem (5) zugeführt, wo Verfahrensschritt (b) vorgenommen wird. Es sei an dieser Stelle betont, daß das Reaktionssystem (3) auch in das Extraktionssystem (5) integriert sein kann.

Das organische Lösungsmittel gelangt über (6) in die Einheit (5). Der gemäß Stufe (b) gewonnene Extrakt wird über Leitung (7) abgezogen. Dieser Extrakt enthält die Percarbonsäure, die der Percarbonsäure entsprechende Carbonsäure, sofern diese in Stufe (a) nicht vollständig umgesetzt wurde, sowie Anteile an Wasser, Wasserstoffperoxid und saurem Katalysator. Der Entfernung dieser drei Verbindungen dienen die beiden nächstfolgenden Verfahrensschritte (c) und (d), die in den Apparaten (8) und (9) durchgeführt werden.

Die Behandlung des gemäß (b) gewonnenen Extraktes mit Wasser oder einer wäßrigen Lösung erfolgt in einer Extraktionseinheit (8), die derart gestaltet ist, daß in ihr 10 theoretische Trennstufen verwirklicht werden können, was durch die eingezeichneten Böden symbolisiert werden soll. Der gewaschene Extrakt verläßt den Apparat (8) über Leitung (10) und wird nachfolgend der Destillationseinheit (9) zugeführt, wo ein Teil des organischen Lösungsmittels zusammen mit dem im Strom (10) — der organischen Lösung der Percarbonsäure — vorliegenden Wasser über Kopf genommen wird. Das Gemisch aus organischem Lösungsmittel und Wasser wird im Kühler (11) kondensiert und im Abscheider (12) in eine organische Phase, die im wesentlichen das organische Lösungsmittel enthält, und in eine wäßrige Phase getrennt. Das in (12) anfallende, organische Lösungsmittel wird beispielsweise über Leitung (13) und (13a) der Extraktionseinheit (8) vor der ersten theoretischen Trennstufe wiederzugeführt.

Es ist aber auch möglich und bei der praktischen Durchführung des Verfahrens vorteilhaft, einen geringen Teil des in (12) anfallenden organischen Lösungsmittels der Kolonne (9) als Rücklauf über (14) wiederzuzuführen. Im allgemeinen ist beim erfindungsgemäßen Verfahren der Anteil, der über (14) nach (9) gelangt, kleiner als der Anteil, der über Leitung (13) und (13a) bzw. (15) nach (8) oder über (13) und (22) nach (5) gegeben wird. Das Mengenverhältnis von Strom (14) zu Strom (13) beträgt beispielsweise 1:10. Bevorzugt liegt dieses Mengenverhältnis im Bereich von 1:5 bis 1:3, ganz besonders bevorzugt im Bereich von 1:3 bis 1:1,1.

Das der Extraktionseinheit (8), also der Verfahrensstufe (c), wiederzuführende organische Lösungsmittel wird nun in der gesamten Menge über (13) und (13a) vor der ersten theoretischen Stufe in (8) eingegeben. Man kann aber auch einen Teil des Stroms (13) über Leitung (15) an einer Stelle der Extraktionseinheit (8) zuführen, die, wie in Fig. 1 angedeutet, hinter der ersten und vor der letzten theoretischen Stufe liegt. Ebenso ist es möglich, den gesamten Strom (13) über Leitung (15) an dieser Stelle einzugeben. Es besteht weiterhin die Möglichkeit, das organische Lösungsmittel, das in (12) anfällt, ganz oder teilweise einer anderweitigen Verwendung zuzuführen und über Leitung (13), (13a) und/oder (15) dann entsprechende Mengen an frischem organischem Lösungsmittel in die Stufe (c), d.h. in die Extraktionseinheit (8) oder über (13) und (22) in die Stufe (b), d.h. in das Extraktionssystem (5), einzuspeisen.

Die im Abscheider (12) auftretende wäßrige Phase wird über Leitung (16) ebenfalls der Einheit (8) zugeführt. Dieser Strom (16) kann gegebenenfalls durch weitere Mengen an Wasser oder wäßriger Lösung über Leitung (17) ergänzt werden.

Die bei der Durchführung von Stufe (c) in der Extraktionseinheit (8) anfallende wäßrige Phase, die den ausgewaschenen sauren Katalysator sowie das zurückgewonnene $H_2O_2$ enthält, wird über Leitung (18) und (18a) entweder der Extraktion (5) oder über Leitung (18) und (19) dem Reaktionssystem (3) zugeführt. Es ist auch möglich einen Teil der wäßrigen Phase, die aus (8) austritt, nach (5) und den verbleibenden Teil nach (3) zu geben. Beispielsweise kann Strom (18) so aufgeteilt werden, daß 40-90 % dieses Stromes in das Reaktionssystem (3) gegeben werden.

Bezüglich der Aufteilung des im Abscheider (12) als organische Phase anfallenden Lösungsmittels auf die einzelnen Mengenströme (14), (13), (13a), (15) und (22) seien beispielsweise folgende Angaben gemacht. Die Angaben beziehen sich auf das in Fig. 1 dargestellte Verfahren. Im Abscheider (12) fallen bei stationärem Zustand der Anlage 150 Teile Essigester sowie 5 Teile Wasser pro Zeiteinheit an. Von dieser Menge an Essigester werden nunmehr 30 Teile über Leitung (14) als Rücklauf auf die Kolonne gegeben, während über Leitung (13) die verbleibenden 120 Teile abgezogen werden.

70 Teile werden z.B. einer im Gegenstrom arbeitenden, aus fünf Mischer-Scheidern bestehenden Einheiten (8) vor dem ersten Mischer zugeführt und 20 Teile Essigester werden hinter dem dritten Mischer-Scheider eingegeben. Die noch verbleibenden 30 Teile werden über (22) der Verfahrensstufe (b) zugeführt. Der aus (5) abgezogene und nach (8) gelangende Extrakt (7) enthält in diesem Fall 180 Teile Essigester.

In einigen Fällen kann es vorteilhaft sein, einen Teil des Stromes (13) nicht der Extraktionseinheit (8), sondern über Leitung (22) der Extraktionsstufe (5), in der Verfahrensschritt (b) vorgenommen wird, zuzuführen. Im allgemeinen jedoch ist der Anteil von (13), der über (22) der Extraktionseinheit (5) eingegeben wird, gering, wenn daneben Teile von (13) über (13a) und/oder (15) nach (8) zurückgeführt werden. Es ist aber auch möglich, die gesamte, in (12) anfallende organische Phase oder einen Teil davon gänzlich nur der Stufe (b), d.h. dem Extraktionssystem (5), zuzuführen, wobei dann keine Beaufschlagung von Extraktion (8) mit in (12) anfallendem organischen Lösungsmittel erfolgt. Bei einer gleichzeitig erfolgenden Rückführung der organischen Phase sowohl nach (8) als auch nach (5) werden im allgemeinen die Mengenverhältnisse der Produktströme so gewählt. daß die Menge von (22) 5-70 % der Menge beträgt, die über (13a) und/oder (15) nach (8) geführt wird. Vorzugsweise wählt man das Mengenverhältnis derart, daß Strom (22) 10-50 % der insgesamt nach (8) gelangenden Menge an organischer Phase beträgt.

Besonders bevorzugt führt man jedoch die gesamte, in (12) anfallende organische Phase oder einen Teil davon entweder gänzlich der Stufe (c) oder gänzlich der Stufe (b) wieder zu.

Bei einer derartigen Arbeitsweise aber, bei der die in (12) anfallende organische Phase ganz oder teilweise sowohl nach (8) als auch nach (5) gegeben wird, wird der Strom (22) bevorzugt ein bis fünf theoretische Trennstufen hinter der Einlaufstelle der über (6) zugeführten organischen Lösungsmittels in die Extraktionseinheit (5) eingegeben.

Das nach (8) und oder (5) zurückgeführte organische Lösungsmittel ist im allgemeinen mit Wasser gesättigt. Es kann daneben auch geringe Menge an Percarbonsäure enthalten, beispielsweise 0,1 bis Gew.-%, die jedoch die Durchführung des Verfahrens nicht beeinflussen. Wird aber ein Teil oder die Gesamtmenge des in (12) anfallenden organischen Lösungsmittels der Verfahrensstufe (b) wiederzugeführt, so ist es vorteilhaft, wenn der Gehalt an Percarbonsäure im Strom (13) bzw. (22) nicht mehr als 1,5 Gew.-% beträgt.

Es sei an dieser Stelle erwähnt, daß sich das Verfahren der Fig. 1, wie für den Fachmann leicht ersichtlich, auf eine Kombination von Percarbonsäure und organischem Lösungsmittel bezieht, wobei der in (8) gewonnene und über (10) abgezogene Extrakt spezifisch leichter ist als die in (8) vorliegende wäßrige, sauren Katalysator enthaltende Phase, die über (18) entnommen wird. Ebenso ist der Strom (7), d.h. die die Verunreinigungen $H_2O$, $H_2O_2$ und starke Säure enthaltende organische Lösung der Percarbonsäure, gegenüber der in (5) auftretenden wäßrigen Phase spezifisch leichter. Das gleiche gilt für die im Abscheider (12) vorliegenden Verhältnisse, wo das organische Lösungsmittel leichter als Wasser ist. Derartige Kombinationen, wo die organischen Phasen sowohl in (5), (8) und (12) spezifisch leichter als die entsprechenden wäßrigen Phasen sind, sind beispielsweise Perisobuttersäure in Benzol, Peressigsäure in Essigsäureäthylester und Perpropionsäure in Diisopropyläther oder in Benzol. Kombinationen, wo die organische Phase im Verfahren in jedem Fall spezifisch schwerer ist als die entsprechenden wäßrigen Phasen, sind bei der Verwendung von Tetrachloräthylen mit den entsprechenden Percarbonsäuren gegeben. Im Falle der Kombination Perpropionsäure und Dichlorpropan (als organisches Lösungsmittel) bildet in der Verfahrensstufe (b) das 1,2-Dichlorpropan bei den in der wäßrigen Phase von (b) vorliegenden Schwefelsäure- und $H_2O_2$-Konzentrationen die leichte Phase, während aber in Stufe (c) die Lösung von Perpropionsäure in Dichlorpropan die schwere Phase darstellt, wenn mit Wasser gewaschen wird. Der Fachmann kann aufgrund der physikalischen Daten der Gemische bzw. der reinen Verbindungen sehr wohl abschätzen, ob die organische Lösung der Percarbonsäure als leichte oder schwere Phase in den einzelnen Verfahrensstufen auftritt. Im übrigen sind derartige Effekte für die erfolgreiche Durchführung des erfindungsgemäßen Verfahrens nicht von Bedeutung; sie erfordern gegebenenfalls lediglich eine umgekehrte Verschaltung der Apparate. In diesem Zusammenhang sei im Hinblick auf die apparative Anordnung ausdrücklich darauf hingewiesen, daß es auch möglich ist, die Extraktionseinheit (8) in die dem Verfahrensschritt (b) dienende Extraktionseinheit (5) zu integrieren. Dies kann bei Anwendung von Extraktionssäulen derart geschehen, daß der Apparat (8) auf die Extraktionssäule (5) aufgesetzt wird und somit eine einzige Säule resultiert, in deren oberen Teil zwischen der n-ten theoretischen und der n-ten + zehnten theoretischen Trennstufe dann der Verfahrensschritt (c) durchführbar ist. Im allgemeinen ist es jedoch günstiger, vorallem wenn für die Wäsche gemäß (c) Wasser eingesetzt wird, eine Batterie von Mischer-Scheidern, die die entsprechende theoretische Trennstufenzahl besitzt, einzusetzen, da dann auch in den meisten Fällen die Eingabe des gegebenenfalls aufgeteilten Stromes (13) definierter erfolgen kann, als dies im allgemeinen bei mit Böden ausgestatteten technischen Extraktionssäulen möglich ist.

Die aus dem Verfahrensschritt (b) kommenden Extrakte (in Fig. 1 der Mengenstrom (7)), für die das Verfahren geeignet ist, enthalten 5 bis 50 Gew.-% Percarbonsäure. Diese Percarbonsäure bildet neben dem organischen Lösungsmittel die Hauptmenge in der Lösung (7). Es ist aber auch möglich, Extrakte einzusetzen, in denen der Carbonsäuregehalt über dem Gehalt an Percarbonsäure liegt. Beispielsweise kann ein Extrakt (7) verwendet werden, der etwa 20 Gew.-% Propionsäure und etwa 15 Gew.-% Perpropionsäure enthält. Im allgemeinen ergibt sich die Konzentration an Percarbonsäure und Carbonsäure im Extrakt aus den in Stufe (a) gewählten Reaktions- bzw. Umsetzungsbedingungen sowie aus der im Schritt (b) angewendeten Menge an organischen Lösungsmittel. Der Gehalt an aus (7) zu entfernenden Verunreinigungen, wie Wasser, $H_2O_2$ und Säurekatalysator, hängt von mehreren Einflußgrößen ab. Einmal spielt die für die Stufe (a) gewählte Konzentration an starker Säure eine Rolle. Daneben ist die Menge an organischem Lösungsmittel im Verhältnis zum Reaktionsgemisch (4) von Bedeutung ebenso wie der $H_2O_2$- bzw. Wassergehalt in diesem das Reaktionssystem (3) verlassenden Produktstrom.

Im allgemeinen werden für das erfindungsgemäße Verfahren aus (5) ablaufende Extrakte eingesetzt, die 0,1 bis 5 Gew.-% Wasser, 0,1 bis 3 Gew.-% Wasserstoffperoxid und 0,05 bis 2 Gew.-% sauren Katalysator, wie beispielsweise Schwefelsäure, enthalten. Selbstverständlich können auch Extrakte eingesetzt werden, in denen beispielsweise der Gehalt an starker Säure bereits weniger als 0,05 Gew.-%, beispielsweise 0,03 Gew.-%, beträgt und der Gehalt an $H_2O_2$ andererseits mehr als 3 Gew.-% beträgt. Die Menge an organischem Lösungsmittel, die von (12) der Stufe (c) oder (b) wiederzugeführt wird, beträgt 10-70 Gew.-% der Menge an organischem Lösungsmittel, die im Strom (7), dem die Verunreinigungen enthaltenden Extrakt, vorliegt.

Bei einigen Kombinationen von Percarbonsäure und organischem Lösungsmittel ist es von Vorteil, nicht die gesamte, nach Schritt (c) zurückgeführte Menge an organischem Lösungsmittel der Einheit (8) vor der ersten theoretischen Trennstufe zuzugeben, sondern einen Teil dieser Menge der Extraktionseinheit (8) an einer Stelle einzugeben, die hinter der ersten und vor der letzten theoretischen Trennstufe liegt.

So ist es beispielsweise (z.B. im System Perisobuttersäure-Benzol) bei Verwendung von vier Mischer-Scheidern als Extraktionseinheit (8) günstig, wenn man etwa 30 % der nach (8) zurückgeführten Menge vor dem dritten Mischer zuführt, während die restlichen 70 % dem Extrakt (7) vor Eintritt in die erste Mischer-Scheider-Einheit zugegeben werden.

Das neben dem organischen Lösungsmittel in (12) anfallende Wasser, das der Extraktionseinheit (8) über (16) wiederzugeführt wird, kann bis zu 10 Gew.-%, vorzugsweise bis zu 5 Gew.-%, Percarbonsäure und bis zu 5 Gew.-%, vorzugsweise nicht mehr als 2 Gew.-%, Wasserstoffperoxid enthalten. Zweckmäßigerweise wird diesem Strom vor Eintritt in die Einheit (8) ein Stabilisator, wie beispielsweise Natriumsalze der Phosphor- und Pyrophosphorsäure, zugesetzt.

Die für den Verfahrensschritt (c) benötigte Menge an Waschlösung kann in Bezug auf die Menge an Extrakt (7) oder in Bezug auf die gesamten, in (8) eintretenden organischen Phasen, wie Strom (13a), (15) und (7), in weiten Grenzen schwanken. Sie beträgt beispielsweise, bezogen auf den Extrakt (7) 0,1-10, vorzugsweise 0,3-5, besonders bevorzugt 0,5 bis 3 Vol.-% an Wasser oder an einer wäßrigen Lösung. Im Hinblick auf eine wirtschaftliche Gestaltung des Gesamtverfahrens ist es jedoch notwendig, die insgesamt in die Verfahrensstufe (c) eingeführten Mengen an Wasser möglichst gezing zu halten. Dem stehen aber wiederum die Erfordernisse entgegen, die durch eine möglichst weitgehende Entfernung der im Extrakt (7) enthaltenen Mengen an $H_2O_2$ und saurem Katalysator gegeben sind.

Der Vorteil des erfindungsgemäßen Verfahrens liegt nun, im Vergleich zu bekannten Verfahren, zum einen darin, daß absolut gesehen, d.h. bezogen auf die Menge an Percarbonsäure, die mit Strom (21) als reine, organische Lösung gewonnen wird, der Anteil an Wasser oder wäßriger Lösung, der für die Stufe (c) eingesetzt werden muß, gesenkt werden kann, während gleichzeitig die Rückwaschwirkung, d.h. die Entfernung der Verunreinigungen wie $H_2O_2$ und starke Säure, besser wird, also die Konzentrationen an diesen Verbindungen im Strom (21) -- bei vergleichbarem Percarbonsäure-Gehalt — letztlich niedriger liegen. Dies ist um so überraschender, als man hätte annehmen müssen, daß sich, bedingt durch die mit der Rückführung des organischen Lösungsmittels sich ergebende Verdünnung des zu waschenden Extraktes (7), eine schlechtere Waschwirkung schon bei gleichbleibender Waschwassermenge ergeben würde.

Ein weiterer, bei technischer Durchführung des erfindungsgemäßen Verfahrens nicht unwesentlicher Vorteil ist darin zu sehen, daß der Energiebedarf für den Verfahrensschritt (d) erheblich abgesenkt werden kann, da die in Einheit (9) zu verdampfenden Anteile an Wasser und organischem Lösungsmittel insgesamt gesehen geringer sind als bei bekannten Verfahren, beispielsweise dem der DE-AS 2 519 289 oder DE-OS 2 519 298.

Der die Einheit (8; Stufe c) verlassende Strom (18), der die wäßrige Lösung darstellt, in der das aus (7) ausgewaschene $H_2O_2$ und die in (7) enthalten gewesenen Anteile an saurem Katalysator vorliegen, enthält daneben Percarbonsäure und gegebenenfalls auch die entsprechende Carbonsäure sowie geringe Anteile an organischem Lösungsmittel. Im allgemeinen beträgt die Löslichkeit an organischem Lösungsmittel in dieser wäßrigen Phase (18) 0,5 bis 10 Gew.-%. Enthält der Strom (18) weniger als 5 Gew.-% an organischem Lösungsmittel, so wird er vorteilhafterweise der Reaktionsstufe (a) wieder zugeführt. Strom (18) kann aber auch, wie bereits erwähnt, ganz oder teilweise der Verfahrensstufe (b) zugeführt werden.

Der Gehalt an Wasser in der die Stufe (c) verlassenden organischen Phase (Strom (10)) beträgt im allgemeinen 0,5 bis 10 Gew.-%, vorzugsweise 1-8 Gew.-%, ganz besonders bevorzugt 1,5-5 Gew.-%. Diese wasseranteile werden in (9) azeotrop über Kopf genommen, so daß der Gehalt an Wasser in Dem Sumpfprodukte der Destillationseinheit (9) (Strom (21)) im allgemeinen unter 1 Gew.-%, vorzugsweise unter 0,5 Gew.-%, besonders bevorzugt unter 0,2 Gew.-% liegt. es ist aber auch möglich, kleinere oder größere Wasserkonzentrationen in der organischen Lösung der Percarbonsäure (21) einzustellen. Bevorzugt wird man jedoch die Einheit (9) so betreiben, daß im Strom (21) weniger als 0,5 Gew.-% wasser vorliegen.

Die nach dem erfindungsgemäßen Verfahren gewonnenen reinen organischen Lösungen von Percarbonsäure enthalten im allgemeinen weniger als 1 Gew.% Wasserstoffperoxid, bevorzugt weniger als 0,6 Gew.%, ganz besonders bevorzugt weniger als 0,3 Gew.-% $H_2O_2$.

Der Gehalt an starker Säure in den als Sumpfprodukt des Verfahrensschritts (d) erhaltenen reinen Percarbonsäure-Lösungen beträgt im allgemeinen weniger als 0,1 Gew.%, bevorzugt weniger als 0,05 Gew.%, ganz besonders bevorzugt nicht mehr als 0,01 Gew.%.

Eine weitere Möglichkeit der Durchführung der erfindungsgemäßen Verfahrens besteht darin, mit einem Lösungsmittelgemisch zu arbeiten, wobei eines der beiden Lösungsmittel, nachfolgend als zweites Lösungsmittel bezeichnet, die Verfahrensschritte (c) und (d) durchläuft und in der Destillationseinheit (9) zusammmen mit dem in

(10) enthaltenen Wasser über Kopf genommen wird. Nachdem dieses zweite Lösungsmittel über Leitung (23) (vgl. Fig. 1) einmalig zugesetzt worden ist, dient es in Schritt (d) als Wasserschlepper und scheidet sich in (12) als organische Phase ab. Danach wird es erfindungsgemäß der Extraktionseinheit (8) über (13) an den bereits erwähnten Stellen zugegeben. Nach Durchlaufen von (8) erscheint es in (10) und der Cyclus beginnt bei kontinuierlicher Durchführung des Verfahrens von neuem.

Das zweite Lösungsmittel, das sogenannte Hilfslösungsmittel, soll folgenden Anforderungen genügen. Es soll eine ähnliche Dichte haben wie das Lösungsmittel, das für den Verfahrensschritt (b) verwendet wird. Weiterhin soll es eine möglichst geringe Wasserlöslichkeit besitzen und mit Wasser ein Azeotrop bilden. Der Siedepunkt des Hilfslösungsmittels soll möglichst mindesten 10 °C unterhalb des Siedepunktes der Percarbonsäure und des Siedepunktes des in (b) verwendeten organischen Lösungsmittels liegen. Es ist natürlich auch möglich, Teile des über (13) zurückgeführten Hilfslösungsmittels über (22) (vgl. Fig. 1) für den Verfahrensschritt (b) zu verwenden, so daß dann Schritt (b) mit einem Gemisch zweier Lösungsmittel durchgeführt wird. Im System Perpropionsäure/Tetrachloräthylen hat sich als Hilfslösungsmittel, das die Stufen (8) und (9) durchläuft, beispielsweise Tetrachlorkohlenstoff oder Chloroform sehr gut bewährt. Auch Benzol kann in der genannten Kombination von Percarbonsäure mit organischem Lösungsmittel als Hilfslösungsmittel verwendet werden.

Die Betriebsbedingungen, unter denen die Verfahrensschritte gemäß (c) und (d) durchgeführt werden, sind bereits bekannt. Die Einheit (8) wird bei Drücken von 800-1 200 mbar und bei Temperaturen von 10-40 °C betrieben. Die Destillationskolonne (9) betreibt man zweckmäßigerweise bei Unterdruck, um die Percarbonsäure einer möglichst geringen thermischen Belastung auszusetzen. Die in (9) eingegebene organischen Lösung der Percarbonsäure (Strom (10)) kann über Leitung (20) mit einem der bekannten Stabilisatoren versetzt werden.

Die für die Kolonne (9) vorzusehende Trennstufenzahl kann in weiten Grenzen variiert werden. Sie hängt naturgemäß vom jeweils vorliegenden System Percarbonsäure/Lösungsmittel ab. Im allgemeinen wird eine Kolonne verwendet, die für Abtriebs- und Verstärkerteil je 2-10 theoretische Böden besitzt. So eignet sich beispielsweise eine Einheit (9) für das System Perpropionsäure/Benzol, die insgesamt 8 theoretische Stufen besitzt und wo der Einlauf des Stromes (10) oberhalb des vierten theoretischen Bodens erfolgt.

Eine Ausführungsform des erfindungsgemäßen Verfahrens sei im folgenden, z.B. für das System Perisobuttersäure/Benzol, anhand von Fig. 2 erläutert.

Aus Verfahrensstube (b) werden im Kontinuierlichen Betrieb über Leitung (1) pro Zeiteinheit ca. 100-120 Teile eines benzolischen Extraktes abgezogen, der 30-32 Gew.-% Perisobuttersäure, 9-11 Gew.-% Isobuttersäure sowie 2-3 Gew.-% Wasser, etwa 1 Gew.-% Wasserstoffperoxid und 0,4-0,6 Gew.-% Natriumhydrogensulfat enthält. Dieser Extrakt wird der Stufe (c) zugeführt, die aus vier, im Gegenstrom arbeitenden Mischer-Scheidern besteht. Gleichzeitig mit dem Extrakt werden über Leitung (2) 28-30 Teile Benzol, das ca. 1 Gew.-% Perisobuttersäure enthält, in den Mischer der ersten Einheit der Mischer-Scheider-Batterie eingefahren. Strom (2) ist Rückstrom aus der Verfahrensstufe (d). Aus dem Scheider der vierten Extraktionsstufe wird über (3) eine gewaschene, benzolische Lösung entnommen, die die folgende Zusammensetzung besitzt : 22-23 Gew.-% Perisobuttersäure, 7-8 Gew.-% Isobuttersäure, 3-4 Gew.-% Wasser sowie 0,1-0,2 Gew.-% $H_2O_2$. Mengenstrom (3) wird in eine bei 200-500 mbar betriebene Destillationskolonne, die mit 5 bis 8 theoretischen Trennstufen versehen ist, eingegeben. Aus dem Sumpf dieser Kolonne des Verfahrensschrittes (d) werden über Leitung (4) pro Zeiteinheit etwa 100 Teile einer völlig wasserfreien benzolischen Lösung abgezogen, die 28-31 Gew.-% Perisobuttersäure neben etwa 10 Gew.-% Isobuttersäure enthält. NaHSO₄ ist nicht mehr nachweisbar. Diese Lösung enthält nur noch etwa 0,1 Gew.-% Wasserstoffperoxid.

Im Destillatbehälter (5) der genannten Kolonne fallen pro Zeiteinheit neben 5-6 Teilen einer wäßrigen Lösung, die ca. 1 Gew.-% Perisobuttersäure und geringe Mengen an $H_2O_2$ enthält, auch 58 bis 60 Teile Benzol an, wovon etwa 30 Teile über Leitung (6) als Rücklauf auf den Kolonne gegeben werden. Das restliche Benzol wird über (2), wie bereits erwähnt, der Stufe (c) zugeführt. Die in (5) anfallende wäßrige Lösung wird über die Leitung (7) in das Mischorgan des vierten Mischer-Scheiders eingesetzt, das gleichzeitig pro Zeiteinheit über (8) mit 0,5 bis 1 Teil Wasser, das geringe Mengen an Natriumpyrophosphat enthält, beaufschlagt wird. Die als schwere Phase aus (c) austretende wäßrige Lösung, die neben Wasserstoffperoxid und Natriumhydrogensulfat auch etwas Benzol und Perisobuttersäure enthält, wird über Leitung (9) entnommen und dem Verfahrensschritt (a) wiederzugeführt.

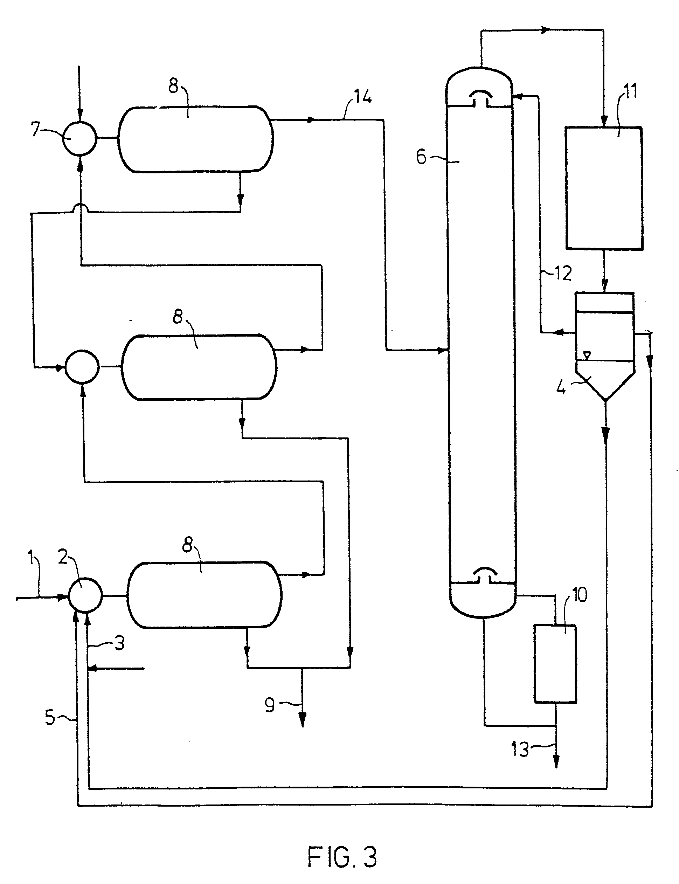

Beispiel 1 (Fig. 3)

Eine durch Extraktion eines aus Schwefelsäure, Wasserstoffperoxid, Propionsäure und Perpropionsäure bestehenden Reaktionsgemisches mit Benzol gewonnene Lösung wird einer Mischer-Scheider-Batterie über Leitung (1) zugeführt. Dieses Extraktionssystem, das bei 30 °C betrieben wird, umfaßt drei übereinander angeordnete Mischer-Scheider, wovon jede Einheit aus jeweils einer Mischpumpe mit nachfolgendem Scheidegefäß (8) von etwa 1,5 l Inhalt besteht.

Die zu behandelnde benzolische Lösung wird in einer Menge von etwa 2 310 g pro Stunde der Mischpumpe (2) der unteren Stufe (8) zugeführt. Diese Lösung enthält neben 22,4 Gew.-% Perpropionsäure und 13,6 Gew.-% Propionsäure die Ver-

unreinigung Wasser, Wasserstoffperoxid und $H_2SO_4$ in der folgenden Konzentration: 0,8 Gew.-%, 0,6 Gew.-% und 0,1 Gew.-%. Der Mischpumpe (2) führt man weiterhin stündlich 90 ml einer wäßrigen Lösung über Leitung (3) zu, die erhalten wird, wenn die Wasserphase (4) des Kopfproduktes der sich anschließenden Azeotropdestillation, die in einer Menge von 69 ml pro Stunde anfällt und neben 4 Gew.-% Perpropionsäure geringe Mengen an $H_2O_2$ enthält, mit 21 ml Wasser mischt, das mit geringsten Mengen Natriumpyrophosphats versetzt ist. Ebenso werden in das Mischorgan (2) über Leitung (5) 822 ml/h Benzol eingegeben, das neben Spuren von Wasser und Propionsäure ca. 1,5 Gew.-% Perpropionsäure enthält. Dieses Benzol ist die organische Phase des Kopfproduktes der Destillationseinheit (6).

Die benzolische Lösung der Perpropionsäure, die dem unteren Scheidegefäß (8) als leichte Phase entnommen wird, führt man nach dem Durchlaufen der mittleren Mischer-Scheider-Anordnung zusammen mit 34 ml Wasser der zu oberen Einheiten gehörenden Mischpumpe (7) zu. Die nach erfolgter Phasentrennung im oberen Schreider (8) anfallende wäßrige Phase wird in die mittlere Extraktionsstufe geleitet. Die in dem mittleren und unteren Scheidegefäß sich als schwere Phasen sammelnden wäßrigen Lösungen werden vereinigt und über Leitung (9) dem Reaktionssystem zugeführt. Die Lösung (9) enthält etwa 32 Gew.-% Perpropionsäure, 16,7 Gew.-% Propionsäure sowie 6,5 Gew.-% $H_2O_2$, 1 Gew.-% Schwefelsäure und geringe Mengen an Benzol. Sie fällt in einer Menge von 190 g pro Stunde an.

Die aus dem oberen Scheider (8) ablaufende benzolische Lösung der Perpropionsäure wird über Leitung (14) in die Destillationseinheit (6) eingeführt. Diese Destillationseinheit besteht aus einer mit 10 Glockenböden versehenen Kolonne von 5 cm Durchmesser, einem Fallfilmverdampfer (10), einem Kondensator (11) sowie einem Abscheider zur Phasentrennung des Destillates. Die Zugabe der aus dem oberen Scheider (8) entnommenen benzolischen Lösung erfolgt aus dem 5. Boden. Bei einem Druck von 400 mbar und einer Temperatur am Kopf der Kolonne von 45-47 °C werden pro Stunde 69 ml wäßrige Phase (4) und 1 274 g Benzol als Destillat erhalten. Von der anfallenbenzolischen Phase werden 625 ml/h als Rücklauf über Leitung (12) auf die Kolonne gegeben, während der Rest der Mischpumpe (2) über Leitung (5) zugeführt wird.

Als Sumpfprodukt dieser Azeotropdestillation wird über Leitung (13) die reine benzolische Perpropionsäure in einer Menge von stündlich 2 172 g aus der Kolonne (6) abgezogen.

Diese Lösung der Perpropionsäure enthält 20,73 Gew.-% PPS (= 5 Mol/h). Der Wassergehalt der Lösung liegt unter 0,05 Gew.-%. Die Konzentration an $H_2O_2$ beträgt 0,1 Gew.-%, während der Gehalt an $H_2SO_4$ unter 5 ppm liegt.

Um die Vorteile des erfindungsgemäßen Verfahrens zu verdeutlichen, werden im folgenden die Zahlen des Beispiels 3 der DOS 2 519 298 zum Vergleich herangezogen, wo ohne Rückführung der organischen Phase des Destillats der Azeotropkolonne gearbeitet wird, d.h. diese Phase vollständig als Rücklauf auf die Azeotropkolonne gegeben wird. Alle Angaben werden auf 5 Mol/h Perpropionsäure, die als Sumpfprodukt der Destillationseinheit (6) erhalten werden, umgerechnet.

Für den Waschvorgang in der dreistufigen Mischer-Scheider-Batterie werden für 5 Mol/h gewonnener benzolischer Perpropionsäure jeweils an Wasser oder einer wäßrigen Lösung benötigt:

| erfindungsgemäß | gemäß DOS 2 519 298 |
|---|---|
| 125 g/h | 149,2 g/h |

Es wird deutlich, daß eine um 16 % verringerte Wassermenge gemäß dem Verfahren der Erfindung, verglichen mit dem der DE-OS 2 519 298, angewendet wird.

Der Gehalt an freiem Wasserstoffperoxid in der benzolischen Lösung der Perpropionsäure (PPS) beträgt beim Verfahren der DE-OS 2 519 298 0,16 Gew.-%. Das bedeutet einen Verlust an $H_2O_2$, der bei 3,55 g pro 5 Mol PPS liegt. Beim erfindungsgemäßen Verfahren beträgt dieser Wert nur 2,2 g/5 Mol PPS.

Besonders deutlich wird die vorteilhafte Arbeitsweise des erfindungsgemäßen Verfahrens, wenn man die in der Destillationseinheit (6) zu verdampfenden Mengen an Wasser und Benzol vergleicht. Beim Verfahren der DE-OS 2 519 298 sind dies 1 740 g/h Benzol, das als Rücklauf auf die Kolonne gelangt, sowie 94 g/h Wasser, jeweils wieder bezogen auf 5 Mol/h benzolischer PPS.

Beim Verfahren gemäß der Erfindung betragen die entsprechenden Werte 1 274 g/h Benzol (Summe aus Mengenstrom (5) und (12)) und 68,8 g/h Wasser.

Weiterhin muß es als Vorteil angesehen werden, daß der Mengenstrom (9), der aus der Mischer-Scheider-Batterie als schwere Phase abläuft, gegenüber der Arbeitsweise der DE-OS 2 519 298 wesentlih verringert werden konnte. Dies ist insofern günstig als die Anteile an Wasser in diesem Strom an anderer Stelle im Verfahren, beispielsweise in der Aufkonzentriereinheit für den sauren Katalysator, wieder ausgetragen werden müssen.

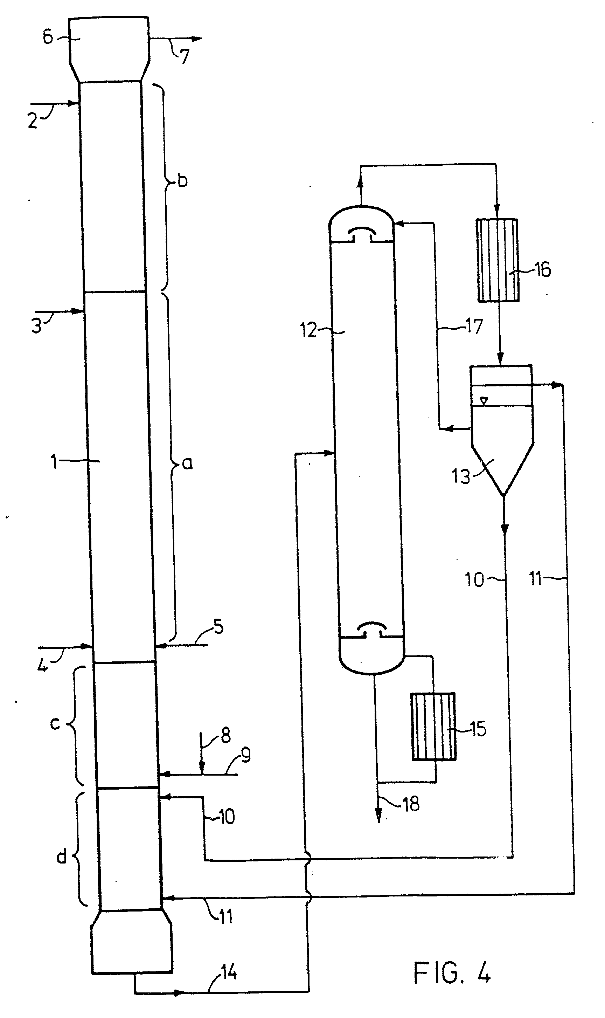

Beispiel 2 (Fig. 4)

In der Extraktionseinheit (1) werden die Umsetzung von Propionsäure mit $H_2O_2$, die Extraktion mit Tetrachloräthylen sowie die Wäsche des Extraktes gleichzeitig vorgenommen.

Der Apparat (1) besteht aus einer pulsierten, mit 200 Siebböden versehenen Kolonne von 2,5 cm Durchmesser. Auf je 1 m Bauhöhe befinden sich etwa 10 dieser Böden, die je nach den über die Höhe variierenden Belastungsverhältnissen mit unterschiedlichem freien Querschnitt ausgestattet sind. Der gesamte Apparat (1) wird auf 30 °C gehalten. Er besitzt jeweils am oberen und unteren Ende ein Abscheidegefäß, in dem die Trennung der Phasen erfolgt.

In der mit (a) bezeichneten Zone werden etwa 9 theoretische Trennstufen verwirklicht. In dieser Zone erfolgt die Umsetzung und die Gewinnung des Extraktes, einer Lösung von Perpropionsäure in Tetrachloräthylen. In Zone (b), die etwa 5 theoretische Stufen besitzt, wird das aus (a) ablaufende Raffinat, das spezifisch leichter ist als das organische Lösungsmittel, mit frischem Tetrachloräthylen behandelt, um Reste von Perpropion- und Propionsäure aus der schwefelsauren Lösung zu entfernen. Zone (b) dient also der Rückwäsche der wäßrigen Phase, während in Zone (c) und (d) die Behandlung des Extraktes mit verdünnter $H_2SO_4$ und Wasser vorgenommen wird. In (c) und (d) können jeweils etwa 3 theoretische Trennstufen realisiert werden.

Bei einer kontinuierlichen Versuchsdurchführung werden der Säule (1) über Leitung (2) pro Stunde 188 ml Tetrachloräthylen eingegeben, das weniger als 0,2 Gew.-% Propionsäure enthält. Über (3) werden stündlich 2,2 kg einer 25 Gew.-%igen Lösung von Propionsäure in Tetrachloräthylen zugeführt, während über (4) und (5) Wasserstoffperoxid und Schwefelsäure in die Kolonne (1) gelangen. Es werden 243 g $H_2O_2$ als 70 Gew.-%ige, wäßrige Lösung (= 5 Mol/h $H_2O_2$) und 280 g einer 75 Gew.-%igen Lösung von Schwefelsäure eingesetzt.

Aus dem am Kopf der Säule befindlichen Scheidegefäß (6) wird über Leitung (7) stündlich eine wäßrige Lösung (483 g) abgezogen, die neben 44-45 Gew.-% Schwefelsäure nur noch etwa 3 Gew.-% $H_2O_2$ enthält.

Über Leitung (8) gelangen 7 g/h 98 Gew.-%iger $H_2SO_4$ und über (9) 28 ml/h Wasser in die Kolonne (1).

Mengenstrom (10) und (17) sind Rückführungen an Tetrachlorkohlenstoff, der als Hilfslösungsmittel für die in Einheit (12) erfolgende azeotrope Entwässerung eingesetzt wird, und an einer wäßrigen Lösung, die dem Abscheider (13) als leichte Phase entnommen wird. Strom (10) ist eine 0,7 Gew.-%ige Lösung von Perpropionsäure in $CCl_4$ und wird mit einer Menge von 685 g/h in die Kolonne eingegeben. Strom (11) ist eine wäßrige Lösung, die neben 2,1 Gew.-% Perpropionsäure noch etwa 0,8-1,0 Gew.-% $H_2O_2$ enthält. Sie fällt in (13) in einer Menge von 93 g/h an und wird vollständig am Fuß der Kolonne (1) eingespeist.

Aus der Säule (1) werden über (14) stündlich 3,35 kg einer 12,3 Gew.-%iger Lösung von Perpropionsäure abgezogen und der nachfolgenden Destillationseinheit (12) zugeführt.

Kolonne (12) ist mit 9 Glockenböden versehen und besitzt einen Durchmesser von 5 cm. Die Beheizung erfolgt mittels des Wärmeaustausches (15), während der Kühler (16) zur Kondensation der über Kopf gehenden Produkte dient. Die Kolonne (12) wird bei einem Druck von 480 mbar betrieben. Die Kopftemperatur liegt bei 47-49 °C. Die Beaufschlagung mit Strom (14) erfolgt oberhalb des 5. Bodens.

Nach Kondensation der Kopfdämpfe fallen im Abscheider (13) stündlich 93 g der wäßrigen Phase und insgesamt etwa 1 355 ml $CCl_4$ an. Von dieser Menge an Tetrachlorkohlenstoff werden über die Leitung (10) die bereits erwähnte Menge abgezogen und der Extraktionssäule (1) wiederzugeführt, während der Rest über Leitung (17) der Kolonne (12) als Rücklauf eingegeben wird.

Aus dem Sumpf der Kolonne (12) werden pro Stunde über Leitung (18) 2 575 g einer 15,7 Gew.-%igen Lösung von Perpropionsäure in Tetrachloräthylen ausgetragen. Diese Lösung enthält kein Wasser und keinerlei Schwefelsäure mehr. Die Konzentration an Propionsäure beträgt 8,4 Gew.-%. Der Gehalt an freiem Wasserstoffperoxid liegt unter 0,1 Gew.-%.

## Ansprüche

1. Verfahren zur kontinuierlichen Herstellung von reinen organischen Lösungen von Percarbonsäuren mit 2 bis 5 Kohlenstoffatomen durch

a) Umsetzung von wäßrigem Wasserstoffperoxid mit 2 bis 5 Kohlenstoffatome enthaltenden Carbonsäuren in Gegenwart saurer Katalysatoren bei einem Einsatzmolverhältnis von $H_2O_2$ zu Carbonsäure wie 0,5 bis 30 : 1,

b) Gewinnung eines im wesentlichen die Percarbonsäure sowie gegebenenfalls die entsprechende, in Stufe (a) nicht umgesetzte Carbonsäure enthaltenden Extraktes durch Extraktion der gemäß Stufe (a) erhaltenen Reaktionsmischung mit einem organischen Lösungsmittel.

c) Behandlung des Extraktes in einer mit ein bis zehn theoretischen Trennstufen versehenen Extraktionseinheit mit Wasser oder einer wäßrigen Lösung und

d) Entwässerung der gemäß (c) erhaltenen organischen Lösung der Percarbonsäure, gegebenenfalls nach Zusatz eines weiteren organischen Lösungsmittels, durch azeotrope Destillation, dadurch gekennzeichnet, daß man die bei der Entwässerung gemäß (d) anfallende organische Phase des Kopfprodukts der Azeotropdestillation ganz oder teilweise in die Stufe (c) und/oder ganz oder teilweise in die Stufe (b) zurückführt.

2. Verfahren nach Anspruch 1, dadurch gekennzeichnet, daß man die gesamte Menge oder einen Teil der in die Stufe (c) zurückzuführenden organischen Phase der für Schritt (c) verwendeten Extraktionseinheit vor der ersten theoretischen Trennstufe zuführt oder an einer Stelle in die für Stufe (c) benutzte Extraktionseinheit eingibt, die sich hinter der ersten und vor der letzten theoretischen Trennstufe befindet.

3. Verfahren nach Anspruch 1 und 2, dadurch gekennzeichnet, daß die organische Phase der Kopfprodukts der Azeotropdestillation der Stufe (d) im wesentlichen aus einem organischen Lösungsmittel mit einem Siedepunkt bei Normaldruck von 30 bis 140 °C besteht.

4. Verfahren nach Anspruch 1 bis 3, dadurch gekennzeichnet, daß die organische Phase des Kopfprodukts der Azeotropdestillation der Stufe (d) im wesentlichen aus einem organischen Lösungsmittel besteht, das ein Azeotrop mit Wasser zu bilden in der Lage ist, dessen Siedepunkt mindestens 10 °C unterhalb des Siedepunkts der Percarbonsäure liegt.

5. Verfahren nach Anspruch 1 bis 4, dadurch gekennzeichnet, daß die organische Phase des Kopfprodukts der Azeotropdestillation der Stufe (d) im wesentlichen aus einem Lösungsmittel besteht, das einen aromatischen Kohlenwasserstoff mit 6 bis 10 C-Atomen, einen aliphatischen oder cycloaliphatischen, jeweils 5 bis 12 C-Atome enthaltenden Kohlenwasserstoff, einen 1 bis 10 C-Atome sowie 1 bis 4 Chloratome enthaltenden Kohlenwasserstoff, einen Ester einer 1 bis 5 C-Atome enthaltenden Carbonsäure mit einem 1 bis 8 C-Atome enthaltenden geradkettigen oder verzweigten Alkohol, oder einen 2 bis 10 C-Atome enthaltenden Äther darstellt.

6. Verfahren nach Anspruch 1 bis 5, dadurch gekennzeichnet, daß man die organische Phase aus (d) für die Rückführung so aufteilt, daß die in die Stufe (b) zurückgeführte Menge 5 bis 70 % derjenigen Menge beträgt, die in die Stufe (c) zurückgeführt wird.

7. Verfahren nach Anspruch 1 bis 6, dadurch gekennzeichnet, daß die Menge der zurückzuführenden organischen Phase 10 bis 70 Gew.-% der Lösungsmittelmenge beträgt, die in dem in Stufe (b) erhaltenen und der Stufe (c) zugeführten Extrakt vorliegt.

8. Verfahren nach Anspruch 1 bis 7, dadurch gekennzeichnet, daß man eine reine organische Lösung von Peressigsäure, Perpropionsäure, Per-n-Buttersäure oder Perisobuttersäure herstellt.

9. Verfahren nach Anspruch 1 bis 8, dadurch gekennzeichnet, daß man reine organische Lösungen von Percarbonsäuren herstellt, die weniger als 0,5 Gew.-% Wasser, weniger als 0,6 Gew.-% Wasserstoffperoxid und weniger als 0,05 Gew.-% sauren Katalysator enthalten.

10. Verfahren nach Anspruch 1 bis 9, dadurch gekennzeichnet, daß man die in Stufe (c) anfallende wässrige Lösung ganz oder teilweise in die Extraktion gemäß Stufe (b) oder ganz oder teilweise in die Umsetzung gemäß Stufe (a) zurückführt.

### Claims

1. Process for the continuous preparation of pure organic solutions of percarboxylic acids having 2 to 5 carbon atoms, by

a) reaction of aqueous hydrogen peroxide with carboxylic acids containing 2 to 5 carbon atoms in the presence of acid catalysts at a feed molar ratio of $H_2O_2$ to carboxylic acid of 0.5 : 1 to 30 : 1,

b) isolating an extract essentially containing the percarboxylic acid and possibly the corresponding carboxylic acid not converted in stage (a), by extracting the reaction mixture obtained according to stage (a) with an organic solvent,

c) treating the extract with water or an aqueous solution in an extraction unit provided with one to ten theoretical separation stages and

d) dehydrating the organic solution of the percarboxylic acid, obtained according to (c), if necessary after adding a further organic solvent, by azeotropic distillation, characterised in that the organic phase of the top product of the azeotropic distillation, obtained during the dehydration according to (d), is entirely or partially recycled to stage (c) and/or entirely or partially recycled to stage (b).

2. Process according to Claim 1, characterised in that the entire amount, or a part, of the organic phase to be recycled to stage (c) is fed to the extraction unit, employed for step (c), upstream of the first theoretical separation stage, or is fed to the said unit at a point downstream of the first, and upstream of the last, theoretical separation stage.

3. Process according to Claim 1 and 2, characterised in that the organic phase of the top product of the azeotropic distillation of stage (d) consists essentially of an organic solvent having a boiling point, under normal pressure, of 30 to 140 °C.

4. Process according to Claims 1 to 3, characterised in that the organic phase of the top product of the azeotropic distillation of stage (d) consists essentially of an organic solvent which is capable of forming an azeotrope, with water, the boiling point of which is at least 10 °C below the boiling point of the percarboxylic acid.

5. Process according to Claims 1 to 4, characterised in that the organic phase of the top product of the azeotropic distillation of stage (d) essentially consists of a solvent which is an aromatic hydrocarbon having 6 to 10 C atoms, an aliphatic or cycloaliphatic hydrocarbon containing 5 to 12 C atoms, a chlorohydrocarbon containing 1 to 10 C atoms and 1 to 4 chlorine atoms, an ester of a carboxylic acid, containing 1 to 5 C atoms, with a straight-chain or branched alcohol containing 1 to 8 C atoms, or an ether containing 2 to 10 C atoms.

6. Process according to Claims 1 to 5, characterised in that the organic phase from (d) is divided up, for recycling, in such a way that the amount recycled to stage (b) is 5 to 70 % of the amount which is recycled to stage (c).

7. Process according to Claims 1 to 6, characterised in that the amount of the organic phase to be recycled is 10 to 70 % by weight of the amount of solvent present in the extract obtained in stage (b) and fed to stage (c).

8. Process according to Claims 1 to 7, characterised in that a pure organic solution of peracetic acid, perpropionic acid, per-n-butyric acid or perisobutyric acid is prepared.

9. Process according to Claims 1 to 8, characterised in that pure organic solutions of percarboxylic acids are prepared which contain less than 0,5 % by weight of water, less than 0,6 % by weight of hydrogen peroxide and less than 0,05 % by weight of acid catalysts.

10. Process according to Claims 1 to 9, characterised in that the aqueous solution obtained in stage (c) is recycled entirely or partially to the extraction according to stage (b) or entirely or partially to the reaction according to stage (a).

**Revendications**

1. Procédé pour la préparation continue de solutions organiques pures d'acides percarboxyliques contenant de 2 à 5 atomes de carbone par

a) réaction d'un peroxyde d'hydrogène aqueux avec des acides carboxyliques contenant de 2 à 5 atomes de carbone en présence de catalyseurs acides à un rapport molaire $H_2O_2$/acide carboxylique de 0,5 à 30 : 1 à la mise en œuvre,

b) isolement d'un extrait contenant essentiellement l'acide percarboxylique et le cas échéant l'acide carboxylique correspondant non converti au stade a), par extraction du mélange de réaction obtenu au stade a) à l'aide d'un solvant organique,

c) traitement de l'extrait à l'eau ou à l'aide d'une solution aqueuse dans une unité d'extraction comportant de 1 à 10 étages de séparation théoriques et

d) déshydratation de la solution organique d'acide percarboxylique obtenue en c), le cas échéant après addition d'un autre solvant organique, par distillation azéotropique, ce procédé se caractérisant en ce que l'on recycle la phase organique du produit de tête de la distillation azéotropique obtenue à la déshydratation selon d) en totalité ou en partie au stade c) et/ou en totalité ou en partie au stade b).

2. Procédé selon la revendication 1, caractérisé en ce que l'on envoie la totalité ou une partie de la phase organique à recycler au stade c) à l'unité d'extraction utilisée pour le stade c) avant le premier étage de séparation théorique ou on l'introduit dans l'unité d'extraction utilisée pour le stade c) à un endroit situé derrière le premier étage et avant le dernier étage de séparation théoriques.

3. Procédé selon les revendications 1 et 2, caractérisé en ce que la phase organique du produit de tête de la distillation azéotropique du stade d) consiste essentiellement en un solvant organique bouillant de 30 à 140 °C à pression normale.

4. Procédé selon les revendications 1 à 3, caractérisé en ce que la phase organique du produit de tête de la distillation azéotropique du stade d) consiste essentiellement en un solvant organique capable de former un azéotrope avec l'eau et dont le point d'ébullition se situe à au moins 10 °C au-dessous du point d'ébullition de l'acide percarboxylique.

5. Procédé selon les revendications 1 à 4, caractérisé en ce que la phase organique du produit de tête de la distillation azéotropique du stade d) consiste essentiellement en un solvant du type hydrocarbure aromatique en $C_6$-$C_{10}$, hydrocarbure aliphatique ou cycloaliphatique en $C_5$-$C_{12}$, hydrocarbure contenant de 1 à 10 atomes de carbone et de 1 à 4 atomes de chlore, ester d'acide carboxylique contenant de 1 à 5 atomes de carbone et d'alcool à chaîne droite ou ramifiée contenant de 1 à 8 atomes de carbone ou éther contenant de 2 à 10 atomes de carbone.

6. Procédé selon les revendications 1 à 5, caractérisé en ce que, pour le recyclage, la phase organique provenant de d) est divisée en sorte que la quantité recyclée au stade b) représente de 5 à 70 % de la quantité recyclée au stade c).

7. Procédé selon les revendications 1 à 6, caractérisé en ce que la quantité de phase organique à recycler représente de 10 à 70 % en poids de la quantité de solvant contenue dans l'extrait obtenu au stade b) et recyclée au stade c).

8. Procédé selon les revendications 1 à 7, caractérisé en ce que l'on prépare une solution organique pure d'acide peracétique, d'acide perpropionique, d'acide per-n-butyrique ou d'acide perisobutyrique.

9. Procédé selon les revendications 1 à 8, caractérisé en ce que l'on prépare des solutions organiques pures d'acides percarboxyliques contenant moins de 0,5 % en poids d'eau, moins de 0,6 % en poids de peroxyde d'hydrogène et moins de 0,05 % en poids de catalyseur acide.

10. Procédé selon les revendications 1 à 9, caractérisé en ce que la solution aqueuse obtenue au stade c) est recyclée en totalité ou en partie à l'extraction du stade b) ou en totalité ou en partie à la réaction du stade a).

0 012 972

1/3

FIG.1

FIG.2

1

FIG. 3

FIG. 4